(19) Europäisches Patentamt
European Patent Office
Office européen des brevets

(11) EP 4 513 505 A1

(12) EUROPEAN PATENT APPLICATION

(43) Date of publication:
26.02.2025 Bulletin 2025/09

(21) Application number: 23200006.7

(22) Date of filing: 27.09.2023

(51) International Patent Classification (IPC):
G16H 40/40 (2018.01)

(52) Cooperative Patent Classification (CPC):
G16H 40/40

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.08.2023 PCT/CN2023/114762

(71) Applicant: Koninklijke Philips N.V.
5656 AG Eindhoven (NL)

(72) Inventors:
• XIE, GUAN XIONG
  5656 AG Eindhoven (NL)
• JIN, SHENG
  5656 AG Eindhoven (NL)
• TSAI, Chris
  5656AG Eindhoven (NL)

(74) Representative: Philips Intellectual Property &
Standards
High Tech Campus 52
5656 AG Eindhoven (NL)

(54) **PRIORITIZATION OF DISINFECTION AND/OR STERILIZATION**

(57) A mechanism for determining the priority for sterilizing and/or disinfecting a medical device. An initial priority is defined using device information. A predicted priority is defined using device information and usage information. The initial priority and the predicted priority are processed to determine the priority for the medical device.

FIG. 1

EP 4 513 505 A1

**Description**

FIELD OF THE INVENTION

The present invention relates to the field of medical devices, and in particular to the field of disinfection and sterilization of medical devices.

BACKGROUND OF THE INVENTION

Disinfection and sterilization play a crucial role in preventing the transmission of infectious pathogens through medical devices to medical subject (e.g., patients). Improper cleaning and disinfection of endoscopes, for example, have been linked to around 15 million hospital-acquired infections in the United States annually.
Disinfection of medical devices typically involves thorough cleaning of the medical device to remove organic substances (such as blood, mucus or other bodily fluids) that may adhere to the devices. Sterilization usually refers to the complete elimination or deactivation of microorganisms, targeting the eradication of any identifiable living microorganisms on a medical device. Sterilization technologies therefore aim to kill, eliminate or otherwise render inert spores, cells, or other reproductive structures present on a medical device.
There is therefore a clear need for a productive and reliable disinfection and sterilization system and/or workflow, to avoid or mitigate the chances that contaminated or unsterile medical devices are used on a medical subject.

SUMMARY OF THE INVENTION

The invention is defined by the claims.
According to examples in accordance with an aspect of the invention, there is provided a computer-implemented method for calculating a final priority of a medical device for disinfection and/or sterilization.
The computer-implemented method comprises: obtaining device information that identifies the medical device; processing the device information to generate an initial priority of the medical device; obtaining usage information indicating a historic and/or future usage of the medical device; processing the device information and the usage information, using a machine-learning method, to generate a predicted priority; and processing at least the initial priority and the predicted priority to calculate the final priority of the medical device.
The present invention thereby provides a mechanism for determining a priority of a medical device that takes account of both static information (the device information) and dynamic information (the usage information) for the medical device. In particular, an initial priority is determined using the static information, which effectively represents a usage independent priority for the medical device. A predicted priority is also determined, which effectively represents a usage dependent priority for the medical device. These two priorities are processed, e.g., combined, to produce and output a final priority for the medical device.
The proposed approach adjusts the calculation of priority based on the unique characteristics and requirements of the medical device, thereby providing a more contextually relevant priority for the medical device. The integration of both static and dynamic information reduces a probability that a medical device will undergo disinfection and/or sterilization at a too late point in time, improving the safety of the medical device.
It has been recognized that a priority for performing disinfection and/or sterilization on a medical device is an important workflow aspect for performing the disinfection and/or sterilization, e.g., to prioritize and/or schedule the performance of disinfection/sterilization. The proposed approach provides a mechanism for establishing a more contextually relevant priority for a medical device.
More particularly, the proposed priority determination algorithm aids in the performance of medical protocol planning and resource allocation, consequently enhancing healthcare efficiency and the quality of patient care.
The computer-implemented method may further comprise obtaining, if available, a user-defined priority for the medical device, wherein the computer-implemented method comprises processing at least the initial priority, the predicted priority and, if available, the user-defined priority to calculate the final priority of the medical device.
In some examples, the computer-implemented method comprises, if the user-defined priority is available, overriding any other calculation of the final priority and setting the user-defined priority as the final priority of the medical device.
The computer-implemented method may comprise, if no user-defined priority is available, combining the initial priority and the predicted priority to calculate the final priority of the medical device.
In some examples, combining the initial priority and the predicted priority comprises performing a weighted sum of the initial priority and the predicted priority.
The processing the device information to generate the initial priority of the medical device may comprise processing the device information using a set of prioritization rules to define the initial priority of the medical device.

The device information may comprise, for each of one or more device parameters, an instance of parameter information.

In some examples, the one or more device parameters comprise a type of the medical device and a category of the medical device.

The step of processing the device information to generate the initial priority of the medical device may comprise: for each device parameter, processing the instance of parameter information to identify a parameter-specific priority of the medical device; and combining all parameter-specific priorities to produce the initial priority of the medical device.

In some embodiments, for each device parameter, processing the instance of parameter information comprises retrieving parameter-specific priority data from a parameter-specific lookup table using the instance of parameter information and processing the parameter-specific priority data to determine the parameter-specific priority.

In some examples, the parameter-specific priority data comprises one or more scores, each score representing a measure of a factor that influences or is influenced by a desire to perform disinfection and/or sterilization on the medical device; and for each device parameter, processing the instance of parameter information comprises processing the one or more scores of the parameter-specific priority data to generate the parameter-specific priority.

Optionally, for each device parameter, processing the instance of parameter information comprises, for each score: retrieving a device-specific weight for the score using the device information; and weighting the score using the device-specific weight.

In some examples, processing the device information and the usage information, using a machine-learning method, to generate a predicted priority comprises: processing the device information and the usage information, using a machine-learning method to generate a desired start and end time for disinfection and/or sterilization; and processing the desired start and end time for disinfection and/or sterilization to produce the predicted priority for the medical device.

There is also provided a computer program product comprising computer program code means which, when executed on a computing device having a processing system, cause the processing system to perform all of the steps of any herein disclosed method. There is also provided a computer-readable (storage) medium comprising instructions which, when executed by a computer, cause the computer to carry out the steps of any herein disclosed method. Preferably, the computer-readable medium is a non-transitory medium. In some examples, there is a provided a data carrier signal carrying the computer program.

There is also provided a processing system for calculating a final priority of a medical device for disinfection and/or sterilization, the processing system being configured to: obtain device information that identifies the medical device; process the device information to generate an initial priority of the medical device; obtain usage information indicating a historic and/or future usage of the medical device; process the device information and the usage information, using a machine-learning method, to generate a predicted priority; and process at least the initial priority and the predicted priority to calculate the final priority of the medical device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a flowchart illustrating a proposed method;
Fig. 2 is a flowchart illustrated an alternative version of a proposed method;
Fig. 3 illustrates a step for a proposed method;
Fig. 4 illustrates additional optional steps for a proposed method; and
Fig. 5 illustrates a proposed processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for determining the priority for sterilizing and/or disinfecting a medical device. An initial priority is defined using device information. A predicted priority is defined using device information and usage information. The initial priority and the predicted priority are processed to determine the priority for the medical device.

In the context of the present disclosure, a priority is a data value that defines an importance or urgency for the medical device to undergo sterilization and/or disinfection (e.g., due to an upcoming need, a risk of failing to disinfect, a difficulty of disinfecting/sterilizing and so on).

The priority may be defined using binary, categorical and/or numeric data. For instance, a priority may be a binary value indicating whether the medical device is "High" or "Low" priority. A categorical priority may indicate a discrete value (e.g., "High", "Medium" or "Low" priority). A numeric value for priority may be defined with respect to a predetermined scale. A predetermined scale may range from 0 to 1, 0 to 10, 1 to 10, 0 to 100 or 1 to 100, although other suitable scales will be apparent to the skilled person.

In the context of the present disclosure, a clinical action or clinical event is an action, procedure or usage of a/the medical device by a clinician, caregiver or target subject in the performance of a clinical task. A clinical task is any task in which the health of the target subject is of consideration. This may include, for instance, an action for treating, transporting, manipulating, testing, analyzing and/or otherwise clinically interacting with a target subject and/or material for the target subject and/or material extracted from the target subject.

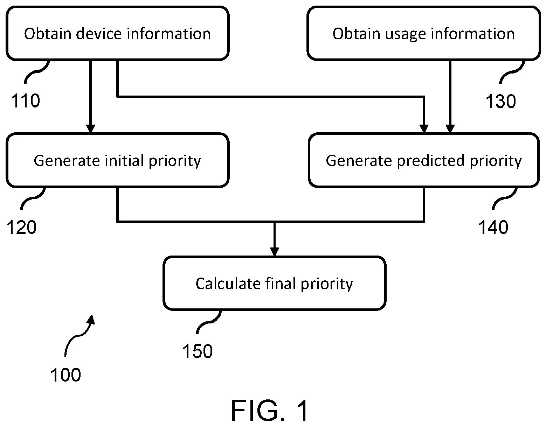

Fig. 1 conceptually illustrates a computer-implemented method 100 according to an embodiment. The method 100 is configured for calculating a final priority of a medical device for disinfection and/or sterilization.

The method 100 may be performed by a processing system. In particular, the processing system may receive or obtain input information, e.g., from a database or other processing system, and output the final priority, e.g., to a further processing system, the database and/or a user interface or display. An example of a suitable processing system is provided later in this disclosure.

The method 100 comprises a step 110 of obtaining device information that identifies the medical device.

Device information provides information usable to identify or distinguish the medical device from other medical devices. More particularly, the device information may be independent of a most recent or historical usage of the medical device, i.e., independent of the occurrence of any (past or future) clinical events or actions using the medical device. Thus, the device information may be independent, uninfluenced or unchanged by the occurrence of any event(s) that necessitate(s) disinfection and/or sterilization.

Put another way, the device information is static information of the medical device that is substantially unchanged between different instances of use of the medical device. The static information may therefore be effectively unchanged between different instances of clinical use of the medical device and/or different instances of sterilization and/or disinfection of the medical device.

The device information may comprise, for each of one or more parameters of the medical device, an instance of parameter information. A non-exhaustive list of examples of device parameters include: identity; type; category; ownership; location; pathology intent; target patient; procedure; and/or timing.

An identify may be a unique identifier for the specific device or the model of device. A type may represent a classification of the medical device by its technical or physical characteristics. A category may represent a classification of the medical device by its application or purpose. An ownership may identify an entity or sub-entity to which the medical device belongs, e.g., classifying to which medical department or individual the medical device belongs. A location may identify a location or intended location of the medical device, e.g., identifying where the medical device is located (e.g., which unit, department or room the medical device belongs. A pathology, if relevant, may identify a pathology or for which the medical device is designed/intended to treat, aid or assist. A target patient may identify a classification of patient for which the medical device is designed/intended to treat, aid or assist (e.g., an identification of age category, gender, weight and so on). A procedure may identify or classify a designed or intended clinical strictness of a procedure of using the medical device (e.g., identifying whether the medical device is to be used invasively or non-invasively). A timing may identify or classify a designed or intended time period during which the medical device is to be used (e.g., a time of day or the like), which recognizes that some medical devices are only used during certain time periods (e.g., a particular fetal ultrasound machine may only be used during the day when ultrasound staff are available).

Accordingly, a non-exhaustive list of example parameter information comprises: type information; category information; ownership information; location information; pathological information; target patient information; procedural information; and/or timing information.

The above provides a variety of examples of different instances of parameter information, each providing information for a different device parameter. Other examples will be readily apparent to the skilled person. Each device parameter for a medical device is associated with a different instance of device information.

By way of example, one medical device may be otherwise identical to another medical device, differing only in their intended time of use (e.g., one is for day patients, with another for night patients), thereby having different timing information.

As another example, one medical device may be otherwise identical to another medical device, differing only in their intended location of use (e.g., one may be assigned to a pediatric unit, with the other assigned to the intensive care unit), thereby having different location and/or ownership information.

The above examples of instances of parameter information demonstrate a sample of the wide variety of information or types of information that can define or delineate the medical device from other forms of medical device. A wide variety of other examples will be readily apparent to the skilled person.

The device information may, for instance, be obtained or retrieved from a database storing device information for a plurality of medical devices. This can be readily performed by a processing system using known data retrieval mechanisms.

The method 100 also comprises a step 120 of generating an initial priority by processing the device information. This effectively produces a priority for disinfection and/or sterilization that is independent of the occurrence of any (past or future) clinical events. In other words, the initial priority is based on static information only. In particular, the initial priority may be calculated by processing only the device information.

The processing of the device information to generate the initial priority of the medical device may comprise processing the device information using a set of prioritization rules to define the initial priority of the medical device. The set of prioritization rules may define or map device information to a priority, e.g., using a decision tree, mapping system or the like. A more detailed alternative example is provided later in this disclosure.

The method 100 also comprises a step 130 of obtaining usage information indicating a historic and/or future usage of the medical device. In particular, the usage information may identify or be responsive to the occurrence or predicted/scheduled occurrence of any clinical events or actions to take place using the medical device. More particularly, the usage information may effectively represent a need, demand or desire for the medical device to be disinfected, e.g., if it is required for an upcoming clinical action and/or has been contaminated by a previous clinical action.

Put another way, the usage information of the medical device is dynamic information that is susceptible to change between different instances of use of the medical device.

Non-exhaustive example content for usage information may include: timing information (e.g., identifying a start and/or end time of a most recent clinical use of the medical device and/or a time period during which the medical device was used (e.g., day of the week)); clinical purpose information (e.g., identifying a purpose, type and/or label for the most recent clinical use of the medical device); user information (e.g., identifying a most recent user of the medical device, e.g., a most recent department, clinician and/or job title of the most recent user of the medical device); and/or DIS&ST information (e.g., identifying a scheduled time for performing disinfection and/or sterilization).

The usage information may comprise any one or more of the identified example information types identified above. However, the above examples of usage information demonstrate a small sample of the wide variety of information or types of information that is responsive or indicative of a usage of the medical device. A wide variety of other examples will be readily apparent to the skilled person.

The usage information may, for instance, be obtained or retrieved from a database storing usage information for a plurality of medical devices. This can be readily performed by a processing system using known data retrieval mechanisms.

The method also comprises a step 140 of processing the device information and the usage information, using a machine-learning method, to generate a predicted priority. The predicted priority effectively represents a priority that is, at least in part, responsive to historic priorities for similar medical devices that have undergone (or will undergo) similar uses.

A machine-learning algorithm is any self-training algorithm that processes input data in order to produce or predict output data. Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

In one example approach, step 140 comprises inputting the device information and the usage information into the machine-learning method to produce a desired start time tsr and a desired end time $t_{end}$ for performing disinfection and/or sterilization. Thus, the machine-learning method may directly output the desired start time $t_{ST}$ and the desired end time $t_{end}$ for performing disinfection and/or sterilization.

In some examples, a desired end time may represent a predicted time at which the medical device is to be reused, or a predetermined period of time before this predicted time. A desired start time may represent a point in time before the desired end time at which disinfection and/or sterilization should be initiated to ensure that the medical device is disinfected and/or sterilized by the desired end time.

When a desired start time and desired end time has been calculated for a plurality of different medical devices, then, for each medical device, a predicted priority $P_{PR}$ can then be calculated using the following equation:

$$P_{PR} = 1 - \frac{(|t_{ST} - t_{smin}| + |t_{end} - t_{emax}|)}{|t_{emax} - t_{smin}|} \qquad (1)$$

where $t_{smin}$ represents the earliest desired start time for performing disinfection and/or sterilization among all medical devices to undergo sterilization and/or disinfection (in the queue). Similarly, $t_{emax}$ represents the latest desired end time for performing disinfection and/or sterilization among all medical devices to undergo sterilization and/or disinfection (in the queue).

In an alternative example approach, step 140 comprises inputting the device information and the usage information into the machine-learning method to directly produce the predicted priority. Thus, the machine-learning method may directly output the predicted priority.

In such examples, in the training dataset for training such a machine-learning algorithm, each training input data entry may comprise an example of device information and usage information with each corresponding training output data entry comprising a predicted priority, e.g., defined by a suitably trained individual and/or extracted from a historic priority for the medical device having the device information and usage information (e.g., as stored in a pre-existing database).

The method 100 also comprises a step 150 of processing at least the initial priority and the predicted priority to calculate the final priority of the medical device. In this way, a usage-independent priority (i.e., static priority) and a usage-dependent priority (i.e., dynamic priority) are combined to produce a final priority of the medical device.

As a simple example, if the initial priority and the predicted priority are numeric, then calculating the final priority may comprise summing or averaging the initial priority and the predicted priority.

As another example, if the initial priority and the predicted priority are numeric, then calculating the final priority may comprise performing a weighted sum or weighted average of the initial priority and the predicted priority. Thus, the initial priority and the predicted priority may be multiplied by a respective (predetermined) weighting value, before the weighted priorities are then summed to produce the final priority.

As another example, if the initial priority and the predicted priority are numeric, then calculating the final priority may comprise performing a multiplication or weighted multiplication of the initial priority and the predicted priority.

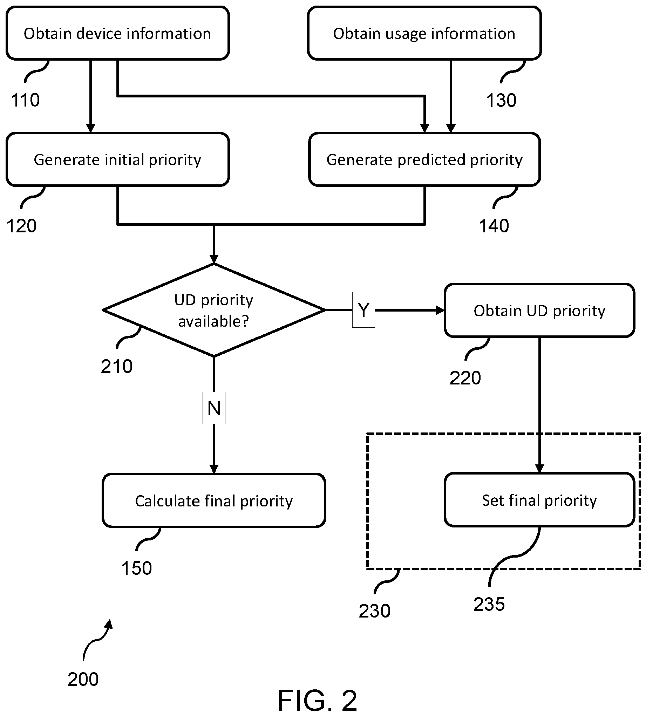

Fig. 2 illustrates another computer-implemented method 200 that acts as a variation to the method previously described method 100.

In this variation, the method comprises obtaining 220, if available, a user-defined priority for the medical device. Thus, the method 200 may comprise a step 210 of determining whether or not a user-defined priority is available. Responsive to a positive determination in step 210, the method 200 moves to the step 220 of obtaining the user-defined priority. Otherwise, the method continues (e.g., moves to the step 150).

If the user-defined priority is available, the method 200 performs a step 230 of processing at least the initial priority, the predicted priority and the user-defined priority to calculate the final priority of the medical device.

In one example, step 230 comprises simply, if the user-defined priority is available, overriding 235 any other calculation of the final priority and setting the user-defined priority as the final priority of the medical device.

Other examples may comprise, if all priorities are numeric, summing or averaging the priorities to produce the final priority. As another example, determining the final priority may comprise performing a weighted sum or weighted average of the priorities to produce the final priority.

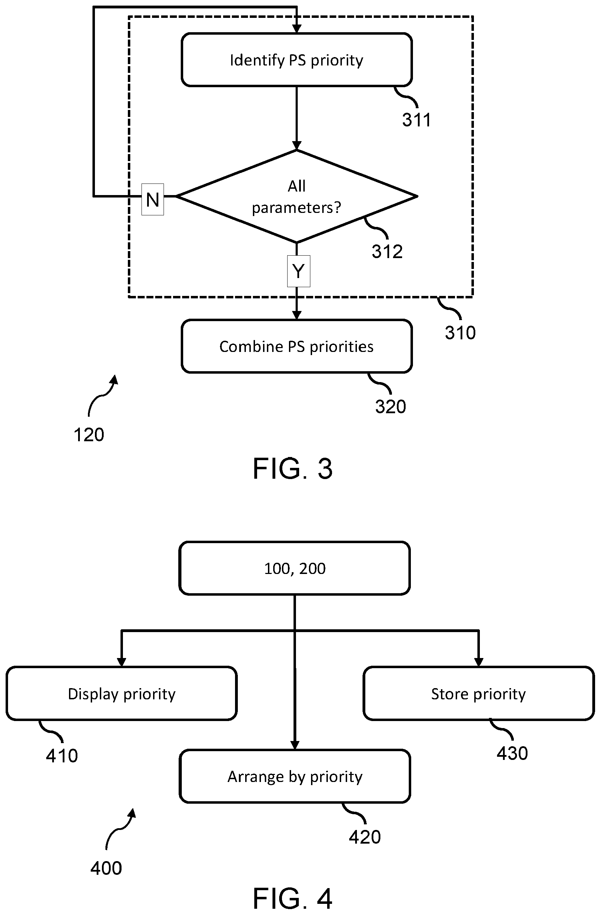

Fig. 3 illustrates an example approach for performing the step 120 of generating an initial priority. In this example, the device information comprises one or more instances of parameter information, each containing information about a different parameter of the medical device.

In the illustrated example, the step 120 comprises a process 310 of for each device parameter, processing the instance of parameter information to identify a parameter-specific priority of the medical device.

Process 310 may, for instance, comprise iteratively identifying 311 a parameter-specific priority for one of the parameters and determining 312 whether a parameter-specific priority has been generated for all parameters (having parameter information in the device information). Responsive to a negative determination in step 312, the process 310 may repeat (with a different or unprocessed parameter). Responsive to a positive determined in step 312, the process 310 ends.

Of course, rather than individually determining the parameter-specific priorities in an iterative process (i.e., one at a time), multiple parameter-specific priorities may be determined in parallel. In some examples, all parameter-specific priorities are determined in parallel.

In the illustrated example, step 120 also comprises a step 320 of combining all parameter-specific priorities to produce the initial priority of the medical device.

In some examples, step 311 comprises retrieving parameter-specific priority data from a parameter-specific lookup table using the instance of parameter information. Thus, a parameter-specific lookup table may define, for a particular parameter, different priority data for different examples of parameter information. The parameter-specific lookup table may be stored or held in a memory or database.

The priority data may define one or more scores. Each score may represent a measure (e.g., a numeric measure) of a factor that influences or is influenced by a desire to perform disinfection and/or sterilization on the medical device.

Example factors include a criticality measure (e.g., representing a measure of criticality or importance of using a device having the instance of parameter information in patient care); a complexity measure (e.g., representing a measure of difficulty or complexity in performing disinfection and/or sterilization on a device having the instance of parameter information); and/or a contamination risk measure (e.g., representing a measure of risk of contamination from a medica device having the instance of parameter information).

Step 311 may also comprise using the device information to weight or modify the score(s) extracted from the parameter-specific lookup table.

In some examples, the device information comprises a device identifier (e.g., a specific code or numeric designation for the device or model of device). The device identifier may be used to identify or otherwise retrieve a device-specific weight for each score.

In particular, the (device identifier of the) device information may define or map to a device-specific weight for each score, e.g., in a device lookup table. The number of device-specific weights will equal the number of scores. The identified device-specific weight may be applied (e.g., multiplied with) the corresponding score to produce a weighted score.

Step 320 may then comprise summing all (weighted) scores to produce the initial priority.

TABLE 1

| Parameter A | Criticality | Complexity | Contamination |
|---|---|---|---|
| A1 | 0.8 | 0.6 | 0.9 |
| A2 | 0.6 | 0.7 | 0.7 |
| ... | ... | ... | ... |
| AN | 0.7 | 0.5 | 0.6 |

Table 1 illustrates an example of a parameter-specific lookup table. The parameter-specific look-up table identifies, for each of a plurality of values (A1, A2, ..., AN) for the parameter ("Parameter A") one or more scores (in the illustrated example: three scores).

TABLE 2

| Device ID | Criticality Weight | Complexity Weight | Contamination Weight |
|---|---|---|---|
| ID1 | 4.2 | 2.8 | 3.6 |
| ID2 | 3.6 | 4.5 | 2.1 |
| ... | ... | ... | ... |
| IDN | 2.9 | 3.2 | 4.8 |

Table 2 illustrates an example of a device lookup table. The device look-up table identifies, for each of a plurality of device identifiers (ID1, ID2, ..., IDN) a respective weight for each score.

Above-described embodiments produce a final priority for a medical device. The skilled person will readily appreciate a wide variety of potential uses for a determined final priority.

Fig. 4 is a flowchart illustrating a variant of additional steps that may be performed using the priority, for the sake of further explanation and example. In particular, Fig. 4 illustrates a method 400 comprising a previously described method 100, 200, together with optional additional steps.

In one example, the method 400 further comprises a step 410 of displaying the determined final priority. Step 410 may, for instance, comprise controlling a user interface to provide a visual representation responsive to the determined final priority. This provides a clinician or other individual a clear indicator of the priority of the medical device. In particular, this enables an individual to directly compare the priorities of different medical devices, to determine an order in which to perform disinfection and/or sterilization of the medical devices.

In some examples, step 410 may also comprise providing a visual representation of one or more other pieces of information generated during the production of the final priority (e.g., the desired start and/or end times where applicable).

In some examples, the method 400 comprises a step 420 of arranging the medical devices by priority. The determined order or arrangement may then be displayed to an individual. This provides useful information for determining or choosing an order for disinfection and/or sterilization.

In some examples, the method 400 comprises a step 430 of storing the final priority for the medical device, e.g., in a database or dataset. In some examples, step 430 may also comprise storing other information generated during the production of the final priority (e.g., the desired start and/or end times where applicable).

The abovementioned method process can be repeatedly performed or implemented for a plurality of medical devices and/or as medical devices are used, sterilized and/or disinfected (and usage information is updated) and/or as new medical devices are made available or added to a dataset to be processed. A repeated priority identification process could be requested by an end user(s) (e.g., clinician) and/or processing unit at any time point after first generating the final priority. This is especially helpful as the usage information is likely to change over time.

In summary, the proposed method and system are designed to establish, for one or more medical devices, an initial priority (based on static information alone), a predicted priority (based on static and dynamic information) and use at least these two priorities to produce a final priority. This final priority can be used to schedule the disinfection and/or sterilization for each medical device, e.g., to ensure the medical devise are ready for future use and/or needs. This reduces the probability that a contaminated medical device will be (e.g., unintentionally) used during a medical procedure. Once the final probability/probabilities have been determined, these can be uploaded to a database for scheduling disinfection and/or sterilization.

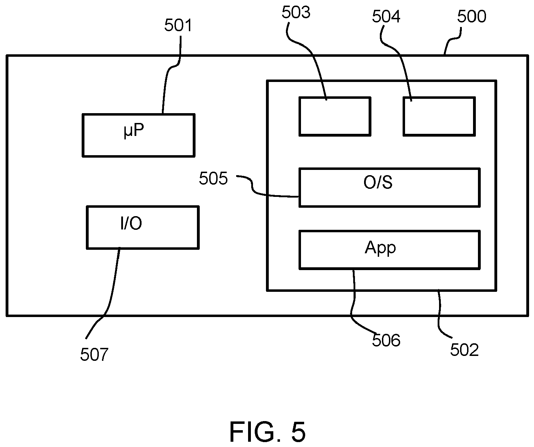

Fig. 5 illustrates an example of a processing system 500 within which one or more parts of an embodiment may be employed.

Various operations discussed above may utilize the capabilities of the processing system 500. For example, one or more parts of a system for generating a final priority (by processing device information and usage information) may be incorporated in any element, module, application, and/or component discussed herein. In this regard, it is to be understood that system functional blocks can run on a single processing system or may be distributed over several processing systems and locations (e.g., connected via internet).

The processing system 500 includes, but is not limited to, PCs, workstations, laptops, PDAs, palm devices, servers, storages, and the like. Generally, in terms of hardware architecture, the processing system 500 may include one or more processors 501, memory 502, and one or more I/O devices 507 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 501 is a hardware device for executing software that can be stored in the memory 502. The processor 501 can be virtually any custom made or commercially available processor, a central processing unit (CPU), a digital signal processor (DSP), or an auxiliary processor among several processors associated with the processing system 500, and the processor 501 may be a semiconductor based microprocessor (in the form of a microchip) or a microprocessor.

The memory 502 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.).

Moreover, the memory 502 may incorporate electronic, magnetic, optical, and/or other types of storage media. Note that the memory 502 can have a distributed architecture, where various components are situated remote from one another, but can be accessed by the processor 501.

The software in the memory 502 may include one or more separate programs, each of which comprises an ordered listing of executable instructions for implementing logical functions. The software in the memory 502 includes a suitable operating system (O/S) 505, compiler 504, source code 503, and one or more applications 506 in accordance with exemplary embodiments. As illustrated, the application 506 comprises numerous functional components for implementing the features and operations of the exemplary embodiments. The application 506 of the processing system 500 may represent various applications, computational units, logic, functional units, processes, operations, virtual entities, and/or modules in accordance with exemplary embodiments, but the application 506 is not meant to be a limitation.

The operating system 505 controls the execution of other computer programs, and provides scheduling, input-output control, file and data management, memory management, and communication control and related services. It is contemplated by the inventors that the application 506 for implementing exemplary embodiments may be applicable on all commercially available operating systems.

Application 506 may be a source program, executable program (object code), script, or any other entity comprising a set of instructions to be performed. When a source program, then the program is usually translated via a compiler (such as the compiler 504), assembler, interpreter, or the like, which may or may not be included within the memory 502, so as to operate properly in connection with the O/S 505. Furthermore, the application 506 can be written as an object oriented programming language, which has classes of data and methods, or a procedure programming language, which has routines, subroutines, and/or functions, for example but not limited to, Python, C, C++, C#, Pascal, BASIC, API calls, HTML, XHTML, XML, ASP scripts, JavaScript, FORTRAN, COBOL, Perl, Java, ADA, .NET, and the like.

The I/O devices 507 may include input devices such as, for example but not limited to, a mouse, keyboard, scanner, microphone, camera, etc. Furthermore, the I/O devices 507 may also include output devices, for example but not limited to a printer, display, etc. Finally, the I/O devices 507 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 507 also include components for communicating over various networks, such as the Internet or intranet.

If the processing system 500 is a PC, workstation, intelligent device or the like, the software in the memory 502 may further include a basic input output system (BIOS) (omitted for simplicity). The BIOS is a set of essential software routines that initialize and test hardware at startup, start the O/S 505, and support the transfer of data among the hardware devices. The BIOS is stored in some type of read-only-memory, such as ROM, PROM, EPROM, EEPROM or the like, so that the BIOS can be executed when the processing system 500 is activated.

When the processing system 500 is in operation, the processor 501 is configured to execute software stored within the memory 502, to communicate data to and from the memory 502, and to generally control operations of the processing system 500 pursuant to the software. The application 506 and the O/S 505 are read, in whole or in part, by the processor 501, perhaps buffered within the processor 501, and then executed.

When the application 506 is implemented in software it should be noted that the application 506 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 506 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions.

A computer program, e.g., embodying the application 506, may be stored/distributed on any suitable computer-readable medium, such as an optical storage medium or a solid-state medium supplied together with or as part of other hardware, but may also be distributed in other forms, such as via the Internet or other wired or wireless telecommunication systems.

In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium. Preferably, the computer readable medium is a non-transitory medium.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A computer-implemented method for calculating a final priority of a medical device for disinfection and/or sterilization, the computer-implemented method comprising:

   obtaining device information that identifies the medical device;
   processing the device information to generate an initial priority of the medical device;
   obtaining usage information indicating a historic and/or future usage of the medical device;
   processing the device information and the usage information, using a machine-learning method, to generate a predicted priority; and
   processing at least the initial priority and the predicted priority to calculate the final priority of the medical device.

2. The computer-implemented method of claim 1, further comprising obtaining, if available, a user-defined priority for the medical device,
   wherein the computer-implemented method comprises processing at least the initial priority, the predicted priority and, if available, the user-defined priority to calculate the final priority of the medical device.

3. The computer-implemented method of claim 2, wherein the computer-implemented method comprises, if the user-defined priority is available, overriding any other calculation of the final priority and setting the user-defined priority as the final priority of the medical device.

4. The computer-implemented of any of claims 1 to 3, wherein the computer-implemented method comprises, if no user-defined priority is available, combining the initial priority and the predicted priority to calculate the final priority of the medical device.

5. The computer-implemented method of claim 4, wherein combining the initial priority and the predicted priority comprises performing a weighted sum of the initial priority and the predicted priority.

6. The computer-implemented method of any of claims 1 to 5, wherein the processing the device information to generate the initial priority of the medical device comprises processing the device information using a set of prioritization rules to define the initial priority of the medical device.

7. The computer-implemented method of any of claims 1 to 6, wherein the device information comprises, for each of one or more device parameters, an instance of parameter information.

8. The computer-implemented method of claim 7, wherein the one or more device parameters comprise a type of the medical device and a category of the medical device.

9. The computer-implemented method of claim 7 or 8, wherein the step of processing the device information to generate the initial priority of the medical device comprises:

   for each device parameter, processing the instance of parameter information to identify a parameter-specific priority of the medical device; and
   combining all parameter-specific priorities to produce the initial priority of the medical device.

10. The computer-implemented method of claim 9, wherein:
    for each device parameter, processing the instance of parameter information comprises retrieving parameter-specific priority data from a parameter-specific lookup table using the instance of parameter information and processing the parameter-specific priority data to determine the parameter-specific priority.

11. The computer-implemented method of claim 10, wherein:

    the parameter-specific priority data comprises one or more scores, each score representing a measure of a factor that influences or is influenced by a desire to perform disinfection and/or sterilization on the medical device; and
    for each device parameter, processing the instance of parameter information comprises processing the one or more scores of the parameter-specific priority data to generate the parameter-specific priority.

12. The computer-implemented method of claim 11, wherein:

for each device parameter, processing the instance of parameter information comprises, for each score: retrieving a device-specific weight for the score using the device information; and weighting the score using the device-specific weight.

13. The computer-implemented method of any of claims 1 to 12, wherein processing the device information and the usage information, using a machine-learning method, to generate a predicted priority comprises:

processing the device information and the usage information, using a machine-learning method to generate a desired start and end time for disinfection and/or sterilization; and
processing the desired start and end time for disinfection and/or sterilization to produce the predicted priority for the medical device.

14. A computer program product comprising computer program code means which, when executed on a computing device having a processing system, cause the processing system to perform all of the steps of the method according to any of claims 1 to 13.

15. A processing system for calculating a final priority of a medical device for disinfection and/or sterilization, the processing system being configured to:

obtain device information that identifies the medical device;
process the device information to generate an initial priority of the medical device;
obtain usage information indicating a historic and/or future usage of the medical device;
process the device information and the usage information, using a machine-learning method, to generate a predicted priority; and
process at least the initial priority and the predicted priority to calculate the final priority of the medical device.

FIG. 1

FIG. 2

Identify PS priority

311

N ── All parameters?

312

Y

Combine PS priorities

310

320

120

## FIG. 3

100, 200

Display priority

Store priority

410

Arrange by priority

430

420

400

## FIG. 4

FIG. 5

**Europäisches Patentamt**
**European Patent Office**
**Office européen des brevets**

# EUROPEAN SEARCH REPORT

Application Number

EP 23 20 0006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2022 0075565 A (EMMA HEALTHCARE CO LTD [KR]) 8 June 2022 (2022-06-08) * paragraphs [0011] - [0079] * ----- | 1-15 | INV. G16H40/40 |
| X | US 2017/135558 A1 (CHOI DAE MYUNG [KR] ET AL) 18 May 2017 (2017-05-18) * paragraphs [0012] - [0065] * ----- | 1-15 | |

| TECHNICAL FIELDS SEARCHED (IPC) |
|---|
| G16H |

The present search report has been drawn up for all claims

| Place of search | Date of completion of the search | Examiner |
|---|---|---|
| Munich | 8 March 2024 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 0006**

This annex lists the patent family members relating to the patent documents cited in the above-mentioned European search report.
The members are as contained in the European Patent Office EDP file on
The European Patent Office is in no way liable for these particulars which are merely given for the purpose of information.

**08-03-2024**

| Patent document cited in search report | Publication date | Patent family member(s) | | Publication date |
|---|---|---|---|---|
| KR 20220075565 A | 08-06-2022 | NONE | | |
| US 2017135558 A1 | 18-05-2017 | CN | 106455963 A | 22-02-2017 |
| | | EP | 3162276 A1 | 03-05-2017 |
| | | JP | 2017524438 A | 31-08-2017 |
| | | KR | 101488786 B1 | 04-02-2015 |
| | | US | 2017135558 A1 | 18-05-2017 |
| | | WO | 2016003052 A1 | 07-01-2016 |

EPO FORM P0459

For more details about this annex : see Official Journal of the European Patent Office, No. 12/82